# EUROPEAN PATENT SPECIFICATION

(11) **EP 0 920 857 B1**
(45) Date of publication and mention of the grant of the patent: **22.11.2006**
(21) Application number: 98500189.0
(22) Date of filing: 10.08.1998
(51) Int. Cl.: A61K 8/27, A61K 8/19, A61K 8/43, A61K 8/49, A61K 33/30, A61K 33/34, A61K 31/155, A61K 31/4425

(54) **Oral composition for the treatment of halitosis**
Orale Zusammensetzung für die Behandlung von übelriechendem Atem
Composition orale pour le traitement de la mauvaise haleine

(30) Priority: 06.11.1997 ES 9702305
(43) Date of publication of application: 09.06.1999
(73) Proprietor: DENTAID, S.A., 08290 Cerdanyola del Vallés (Barcelona) (ES)
(72) Inventor: Masdevall Noguera, Enrique, 08290 Cerdanyola del Valles (ES)
(74) Representative: Ponti Sales, Adelaida

(56) References cited:
- EP-A- 0 321 180
- EP-A- 0 368 130
- WO-A-96/37183
- US-A- 5 320 829

## Description

### FIELD OF THE INVENTION

The present invention relates to a stable oral composition permitting total bioavailability of active ingredients of considerable anti-plaque effect with low staining of the teeth for the treatment of oral halitosis.

The invention also relates to the use of said composition in the form of mouth rinses, dentifrices and the like for the treatment of oral halitosis.

### BACKGROUND OF THE INVENTION

Halitosis is the term used to describe bad breath emitted by the mouth, independently of whether the bad-smelling substances are of oral origin or non-oral origin such as the respiratory or digestive tracts.

The etiology and pathogenesis of oral halitosis is based on local buccal factors which lead to high microbial metabolism in alkaline medium. This causes the emission of volatile sulphur compounds (VSC) responsible for the bad breath, most of which have their origin in the breakdown of sulphur-rich hydrolysed amino acids by anaerobic gram-negative bacteria in an alkaline medium.

The problems of oral halitosis often accompany and can be the manifestation of pathologies of the oral cavity caused, for example, by excessive accumulation of bacterial plaque. Under this aspect there are numerous references to dental compositions with bactericidal and anti-plaque activity.

Patent US-4,022,880 describes the anti-plaque effect provided by combining Zn salts with an antibacterial agent such as chlorhexidine.

However, prolonged utilisation of chlorhexidine digluconate at concentrations higher than 0.20% leads to staining of the teeth and loss of sense of taste. Chlorhexidine presents sustained activity over the course of time.

Furthermore, patent US-4,647,452 relates to an oral composition of improved efficacy against dental plaque. Said oral composition comprises zinc salts with salicylamides of specific structure for use thereof in the inhibition of dental plaque.

Patent EP 0546627-A1 describes a stable oral anti-plaque composition which contains zinc salts and Triclosan in a wetting system. The object of said patent is to provide a stable oral composition with marked anti-plaque activity by combining an anti-plaque system resulting from a mixture of zinc salts with Triclosan, with a wetting agent system resulting from the combination of a high water content with a certain type of surface-active agents.

Patent US-5,236,699 describes an anti-plaque mouth-rinse composition which includes Triclosan (5-chloro-2-(2,4-dichlorophenoxy)-phenol and cetylpyridinium chloride which when used separately are of limited efficacy but, when combined, increase the antibacterial activity, thereby inhibiting the formation of dental plaque. Triclosan alone, however, does not show a long effect over time, due its low substantivity [Gilbert 1987], while its anti-plaque activity is lower than that of chlorhexidine [Jekins, Addy and Newcombe, J. Clin. Periodontol.,1994:21:250-255].

Also forming part of the state of the art is the disclosure of international patent WO 92/13514. Said patent relates to oral compositions for buccal hygiene which include bromochlorophene and zinc ions in a ratio by weight between 1:0.1 and 1:50. The combination of these components provides a synergetic anti-plaque effect.

Also increasingly evident among the aforesaid problems related with teeth care are those involving bad breath from the mouth. Patent EP 0436284-A1 discloses a stable oral composition which includes a zinc compound for the control of bad breath and prevention of calculus.

Patents U.S. 2,894,876 and U.S. 5,211,940 also mention the use of certain Cu(+2) salts in preparations for oral use in attempting to combat bad breath.

The effect of the metallic ions Zn(+2) and or Cu(+2), when used alone and without any other active ingredient, is relatively limited and does not permit highly efficacious treatment of the problem of halitosis.

Therefore, there does not yet exist in the state of the art a stable composition which presents high anti-plaque effect, low staining of the teeth, total bioavailability of the active ingredients, while also presenting marked anti-halitosis activity.

### DESCRIPTION OF THE INVENTION

The composition of the present invention manages to overcome the problems of the prior art, while also providing other advantages which will be described below.

The present invention relates to a stable oral composition of high anti-plaque effect, low staining of the teeth, total bioavailability of the active ingredients and marked antihalitosis activity.

The authors of the present invention have found, surprisingly, that a certain combination of three compounds, two antiseptic agents at very low doses and a salt or compound of Zn(+2) and/or Cu(+2), has an anti-plaque effect and anti-halitosis activity greater than that which could be obtained from the components used separately.

The composition of the present invention includes chlorhexidine digluconate in a concentration by weight of 0.025 to 0.20% or another chlorhexidine salt in an equivalent concentration of chlorhexidine base, cetylpyridinium chloride or another salt of quaternary ammonium in a concentration by weight of 0.025 to 0.10% and a pharmaceutically acceptable salt or compound of Zn(+2) and/or Cu(+2) which includes from 100 to 1,000 ppm of Zn(+2) and/or Cu(+2) ions.

The composition of the present invention does not affect the composition of the bacterial flora present in the oral cavity, and provides high anti-plaque effect with low staining of the teeth.

The composition of the invention provides a product with marked anti-halitosis activity, for daily use and without negative effects due to the low concentration of the active ingredients present in same.

There follows a brief description of the characteristics of each one of the components used in the composition of the present invention.

On the one hand, chlorhexidine digluconate [1,6-bis (*N*⁵-*p*-chlorophenyl-*N*¹-diguanido)hexane digluconate] is a bisbiguanide derivative with a cationic charge. It is a bactericidal and anti-plaque agent of high substantivity to the structures of the oral cavity (teeth enamel, mucous membranes, etc.). It is effective against a broad spectrum of gram-positive and gram-negative bacteria. The chlorhexidine gluconate has water solubility exceeding 50% by weight, with slightly acidic reaction and precipitates in alkaline medium. Chlorhexidine has the property of preventing bacterial adhesion, interrupting the formation of bacterial masses, maintaining an anti-microbial state of activity and retaining its effect over a long period of time (substantivity).

Furthermore, the quaternary ammonium salts usually present intense bactericidal activity, though of shorter duration than that of chlorhexidine. The CPC in particular presents detergent and antiseptic activity, though the presence of proteins, serum, lipids and phospholipids reduces said activity.

The free Zn(+2) and Cu(+2) ions show that they possess great capacity for forming insoluble salts with nucleophilic compounds such as valeric acid, hydrogen sulphide, mercaptans and the like. These compounds are the anaerobic by-products of the gram-negative bacteria and the ones chiefly responsible for bad breath.

In the comparative examples, which will be described below, in the examples section, the surprising effect of the oral composition of the present invention can be observed.

The composition of the invention further includes a wetting agent selected from among glycerine, sorbitol 70%, PEG-400 and PEG-600, propylene glycol or the like in a concentration by weight of 5-15%. The wetting agent is added for three purposes: to lend "body" to a composition which would otherwise be of very low viscosity, to eliminate the possibility of crystallisation and, finally, to add a complementary sweetening effect different from that imparted by the saccharine.

The composition also includes a non-ionic or amphoteric surface-active agent selected from among polyoxyethylene esters (sorbitan-monoisostearate, monoisooleate, monolaurate), copolymers in block of poly(oxypropylene)-poly(oxyethylene), polyhydroxypropyl esters, PEG-40 hydrogenated castor oil, PEG-60 hydrogenated castor oil, cocamido propyl betaine and the like in a concentration by weight of 0.2-0.8%. The surface-active agent is also added with a double purpose: on the one hand, to solubilize the essences present, since they are oily in character and, on the other hand, to lend a certain foaming capacity to the composition and thereby facilitate the suspension and consequent elimination of impurities present in the oral cavity.

It also includes fluorides selected from among sodium fluoride, cetyl-amine fluorhydrate, a derivative fluorinate of octadecylamine (DFO) or another pharmaceutically acceptable source of fluorine ions corresponding to a concentration in free fluorine ions of up to 2500 ppm. The fluorinated compounds are added to the composicion in order to favour remineralisation of the teeth. Fluorides at a low concentration cause the hydroxylapatite Ca₅(PO₄)₃OH to be converted into fluoroapatite Ca₅(PO₄)₃F, which is more resistant to acid attack. Moreover, the fluorides block, among other enzymes, the enzyme enolase which is responsible for conversion of the carbohydrates into acids.

The composition also includes a sweetener such as saccharine at a concentration by weight of 0.005-0.10% and/or xylitol at a concentration by weight of 2-10%, essences in a concentration by weight of 0.05-0.20 and a colorant in a concentration by weight of 0.0001 - 0.001%.

None of the additional components described above is essential for obtaining the surprising effect of the composition of the present invention, though the addition thereof does enhance the properties of the end product.

There follows an outline of the main advantages involved in utilisation of the composition of the present invention. The composition of the invention reduces the anaerobic gram-negative bacteria mainly responsible for the halitosis phenomenon. The products of decomposition are neutralised with immediate reduction of the bad breath and of the irritation which can be provoked by the volatile sulphur compounds, VSC.

Furthermore, owing to the low concentration of antiseptics and especially of chlorhexidine, minimal or nil alteration of the oral flora is achieved, while staining of the teeth and loss of sense of taste is also avoided.

An anti-caries action is achieved when the composition includes the additional fluoride component described above. Furthermore, due to the very good substantivity of the active ingredients used, a prolonged action over time is achieved, which makes frequent applications unnecessary (maximum twice daily).

Provided below are a number of non-restrictive examples of the scope of the invention, showing preferred embodiments of the composition of the invention. Possible variations which an expert in the subject might make in the components of the composition and considered equivalent to the elements described in the description of the invention will also be considered within the sphere of protection of the present invention.

Table A below shows seven compositions which include the different percentages and components described in the description of the invention.

**Table A**

| Component | Comp. 1 (%) | Comp. 2 (%) | Comp. 3 (%) | Comp. 4 (%) | Comp. 5 (%) | Comp. 6 (%) | Comp. 7 (%) |
|---|---|---|---|---|---|---|---|
| Chlorhexidine digluconate | | | | | | | |
| (CHX) | 0.05 | 0.025 | 0.05 | 0.05 | 0.05 | 0.075 | 0.075 |
| cetylpyridinium chloride (CPC) | 0.05 | 0.025 | 0.05 | 0.05 | 0.05 | 0.05 | 0.05 |
| Cu gluconate | | | | | | 0.25 | |
| Zn gluconate | 0.50 | | | 0.50 | | | |
| Zn lactate | | 0.15 | 0.15 | | | | |
| Zn chloride | | | | | 0.15 | | |
| Cu acetate | | | | | | | 0.10 |
| Saccharine | 0.01 | 0.01 | 0.01 | 0.01 | 0.01 | 0.01 | 0.01 |
| Xylitol | 5.0 | 5.0 | 10.0 | 5.0 | 5.0 | 10 | 10 |
| Glycerine USP | 5.0 | 10.0 | 10.0 | 5.0 | 5.0 | 10 | 10 |
| Colorant | 0.0001 | 0.0001 | 0.0001 | 0.0001 | 0.0001 | 0.0001 | 0.0001 |
| PEG-40 hydrogenated | | | | | | | |
| castor oil or | 0.60 | 0.60 | 0.60 | 0.60 | | 0.60 | 0.60 |
| cocamido propyl betaine | | | | | 0.80 | | |
| Essence | 0.15 | 0.10 | 0.15 | 0.15 | 0.20 | 0.15 | 0.15 |
| Alcohol 96% or | 5.0 | | 1.0 | 5.0 | - | - | - |
| 2,4-dichlorobenzyl alcohol | | 0.10 | | | - | - | - |
| Deionized water c.p.s. | 100 | 100 | 100 | 100 | 100 | 100 | 100 |
| pH | 6.30 | 6.15 | 6.20 | 6.30 | 5.90 | 6.15 | 6.0 |

Composition 5 also includes a fluorinated derivative of octadecylamine (DFO) at a concentration of 0.80%, approximately equivalent to 200 ppm of fluorine ions.

Comparative examples have also been carried out showing the surprising effect of the composition of the present invention.

### COMPARATIVE EXAMPLES

In vitro microbiological examples are provided below relating to the antibacterial activity on microorganisms involved in the problem of halitosis, both with respect to the individual active ingredients and the binary mixtures and the composition of the invention.

### Example 1

The anti-microbial activity of a certain number of active ingredients was obtained by study of the minimum inhibitory concentrations (MIC) of the respective agents (by the method of diffusion in agar) against bacteria representing periodontal disease.

The results of this evaluation are shown in Table 1 below.

**TABLE 1**

| Antimicrobial activity (in %) against bacteria | | | |
|---|---|---|---|
| **Agents** | **Bacteria** | | |
| | *P.gingivalis* | *P.intermedia* | *A.actinomycetemcomitans* |
| Zn⁺⁺, sulphate | 0.5 | 0.5 | 0.1 |
| Hexetidine | 0.001 | 0.001 | 0.09 |
| Chlorhexidine | 0.002 | 0.0009 | 0.004 |
| CPC | 0.0006 | 0.0003 | >0.02 |
| Triclosan | 0.004 | 0.009 | 0.0006 |
| Cu⁺⁺ gluconate | 0.06 | 0.004 | NI* |

| | | | |
|---|---|---|---|
| * Not inhibited | | | |

The data show the scant activity of zinc salts and the better activity of the rest of the active ingredients, notable among which is chlorhexidine due to its better results in general on the three bacteria.

### Example 2

The presence or absence of activity in a certain number of binary mixtures of active ingredients was determined by the method of diffusion in agar using discs impregnated in the respective mixtures, versus of major bacteria associated with periodontal disease.

The results are shown in Table 2 below.

**TABLE 2**

| Presence of anti-microbial activity in binary mixtures | | | | |
|---|---|---|---|---|
| | **Mixtures** | *P.gingivalis* | *P.intermedia* | *A.actinomycetemcomitans* |
| 1 | Hexetidine+ Chlorhexidine | + | + | - |
| 2 | Zn⁺⁺ Sulphate + chlorhexidine | + | + | + |
| 3 | CPC + Hexetidine | + | + | - |
| 4 | Zn⁺⁺ Sulphate+ Hexetidine | + | + | + |
| 5 | Zn⁺⁺ Sulphate + CPC | + | + | + |
| 6 | CPC+Chlorhexidine | + | + | + |

The results showed the appearance of strong antagonisms, of which mixtures 1 and 3 are an example, against *A. actinomycetemcomitans*. No notable antagonisms or synergies could be observed for the other cases from the diffusion haloes obtained.

The MICs of the binary mixtures selected were then obtained, against each agent alone, as a control in order to determine any possible increases in the anti-microbial efficacy of the mixtures.
The MIC readings shown in any case gave a very similar MIC to that of the most effective product for each bacterium, which led to the conclusion that there was possibly an "in vitro" potential additive effect of the substances studied.

### Example 3

The anti-microbial activity of the mouth rinse evaluated "in vitro" by mortality studies over short intervals of time (short interval killing test, SIKT) against a mixture of periodontal bacteria containing between 1.0*10⁵ and 1.0*10⁷ CFU ml of each strain. The total counts obtained in the surviving flora and those pertaining to each strain after one minute of contact are summarised in Tables 3 and 4.

**TABLA 3**

| **Total CFU/ml recovered (%) at 1 minute** | |
|---|---|
| PBS | 100% |
| Composition of the inv. | 0.05% |
| CHX | 25.55% |
| Zn⁺⁺, sulphate | 47.65 |
| CPC | 0.04% |

**TABLE 4**

| **CFU/ml recovered from each strain (%)** | | | | | |
|---|---|---|---|---|---|
| | PBS | Composition of the inv. | CHX | Zn⁺⁺, sulphate | CPC |
| *F. nucleatum* | 100% | 0.35 | 0.87 | 40.9 | 0.05 |
| *P. gingivalis* | 100% | <0.01 | <0.81 | 33.9 | <0.01 |
| *P. intermedia* | 100% | <0.01 | <0.56 | 0.56 | 0.01 |
| *Peptostreptococcus micros* | 100% | 0.06 | 82.12 | 54.5 | 0.03 |

From Table 3 it can be deduced that the initial effect of the composition of the invention against the total flora was similar to that of the CPC, which gave viable recovered percentages similar to those of the composition of the invention.

The conclusion that the principal anti-microbial activity of the composition of the invention is due only to the CPC is partly corrected thanks to the data of Table 4. The data shown in Table 4 show and confirm its activity on various bacteria, improved for *P. intermedia* by the presence of CHX and Zn⁺⁺.

## Claims

1. Oral composition cetylpyridinium which includes a) chlorhexidine digluconate in a concentration by weight of 0.025 to 0.20% or another soluble and pharmaceutically acceptable chlorhexidine salt in an equivalent concentration of chlorhexidine base; b) cetylpyridinium chloride or another pharmaceutically acceptable salt of quaternary ammonium in a concentration by weight of 0.025 to 0.10%; c) a pharmaceutically acceptable salt or compound of Zn(+2) and/or Cu(+2) corresponding to a concentration in metallic ions of Zn (+2) and/or Cu (+2) comprised between 100 and 1000 ppm.

2. Composition as claimed in Claim 1, which further includes a wetting agent selected from among glycerine, sorbitol 70%, PEG-400 and PEG-600, propylene glycol or the like in a concentration by weight of 5-15%.

3. Composition as claimed in Claims 1 or 2, which also includes a non-ionic or amphoteric surface-active agent selected from among polyoxyethylene esters (sorbitan-monoisostearate, monoisooleate, monolaurate), copolymers in block of poly(oxypropylene)-poly(oxyethylene), polyhydroxypropyl esters, PEG-40 hydrogenated castor oil, PEG-60 hydrogenated castor oil, cocamido propyl betaine and the like in a concentration by weight of 0.2-0.8%.

4. Composition as claimed in Claim 1, 2 or 3, which also includes fluorides selected from among sodium fluoride, cetyl-amine fluorhydrate, a derivative fluorinate of octadecylamine (DFO) or another pharmaceutically acceptable source of fluorine ions corresponding to a concentration in free fluorine ions of up to 2500 ppm.

5. Composition as claimed in any of the previous claims, which also includes a sweetener such as saccharine at a concentration by weight of 0.005-0.10% and/or xylitol at a concentration by weight of 2-10%.

6. Composition as claimed in any of the above claims, which also includes essences in a concentration by weight of 0.05-0.20 and a colorant in a concentration by weight of 0.0001 - 0.001%.

7. Composition as claimed in any of Claims 1 to 6, for use thereof in the treatment of oral halitosis.

8. Use of the composition claimed in any of Claims 1 to 6 for obtaining a mouth rinse for the treatment of oral halitosis.

9. Use of the composition claimed in any of Claims 1 to 6 for obtaining a toothpaste for the treatment of oral halitosis.

10. Use of the composition claimed in any of Claims 1 to 6 for obtaining a dental powder for the treatment of oral halitosis.

11. Use of the composition claimed in any of Claims 1 to 6 for obtaining a dental chewing gum for the treatment of oral halitosis.

## Patentansprüche

1. Orale Zusammensetzung, die a) Chlorhexidindigluconat in einer Konzentration, bezogen auf das Gewicht, von 0,025 bis 0,20 % oder ein anderes lösliches und pharmazeutisch verträgliches Chlorhexidinsalz in einer äquivalenten Konzentration der Chlorhexidinbase; b) Cetylpyridinchlorid oder ein anderes pharmazeutisch verträgliches Salz von vierwertigem Ammonium in einer Konzentration, bezogen auf das Gewicht, von 0,025 bis 0,10 %; c) ein pharmazeutisch verträgliches Salz oder eine Verbindung aus Zn(+2) und/oder Cu(+2), entsprechend einer Konzentration in Metallionen von Zn(+2) und/oder Cu(+2) umfasst zwischen 100 und 1.000 ppm, einschließt.

2. Zusammensetzung nach Anspruch 1, die weiterhin ein Benetzungsmittel, ausgewählt unter Glycerin, Sorbitol 70%, PEG-400 und PEG-600, Propylenglykol oder ähnlichem in einer Konzentration, bezogen auf das Gewicht, von 5-15 % einschließt.

3. Zusammensetzung nach Anspruch 1 oder 2, die auch ein nicht-ionisches oder amphoterisches oberflächenaktives Mittel, ausgewählt unter Polyoxyethylen Estern (Sorbitan-Monoisostearat, -Monoisooleat, -Monolaurat), Copolymeren im Block von Poly(oxypropylen)-poly(oxyethylen), Polyhydroxypropyl Estern, PEG-40 hydrogeniertern Rizinusöl (Castoröl), PEG-60 hydrogeniertern Rizinusöl (Castoröl) und Cocamidpropylbetain und ähnlichem in einer Konzentration, bezogen auf das Gewicht, von 0,2-0,8 % einschließt.

4. Zusammensetzung nach einem der Ansprüche 1, 2 oder 3, das auch Fluoride, ausgewählt unter Natriumfluorid, Cetylamin Fluorhydrat, einem Derivat Fluorinat von Oc-tadecylamin (DFO) oder einer anderen pharmazeutisch verträglichen Quelle von Fluorionen entsprechend einer Konzentration an freien Fluorionen von bis zu 2.500 ppm einschließt.

5. Zusammensetzung nach einem der vorhergehenden Ansprüche, die auch einen Süßstoff, wie Saccharin in einer Konzentration, bezogen auf des Gewicht, von 0,005-0,10 % und/oder Xylitol in einer Konzentration, bezogen auf des Gewicht, von 2-10 % einschließt.

6. Zusammensetzung nach einem der obigen Ansprüche, die auch Essenzen in einer Konzentration, bezogen auf das Gewicht, von 0,05-0,20 und ein Färbemittel in einer Konzentration, bezogen auf das Gewicht, von 0,0001-0,001 % einschließt.

7. Zusammensetzung nach einem der Ansprüche 1 bis 6 zur Verwendung in der Behandlung von Mundgeruch.

8. Verwendung der Zusammensetzung nach einem der Ansprüche 1 bis 6 zum Erhalten einer Mundspülung für die Behandlung von Mundgeruch.

9. Verwendung der Zusammensetzung nach einem der Ansprüche 1 bis 6 zum Erhalten einer Zahnpasta für die Behandlung von Mundgeruch.

10. Verwendung der Zusammensetzung nach einem der Ansprüche 1 bis 6 zum Erhalten eines Dentalpulvers für die Behandlung von Mundgeruch.

11. Verwendung der Zusammensetzung nach einem der Ansprüche 1 bis 6 zum Erhalten eines zahnmedizinischen Kaugummis für die Behandlung von Mundgeruch.

## Revendications

1. Composition orale qui comprend a) du digluconate de chlorhexidine avec une concentration en poids de 0,025 à 0,20% ou un autre sel de chlorhexidine soluble et pharmaceutiquement acceptable avec une concentration équivalente de chlorhexidine base; b) du chlorure de cétylpyridinium ou un autre sel pharmaceutiquement acceptable d'ammonium quaternaire avec une concentration en poids de 0,025 à 0,10%; c) un sel pharmaceutiquement acceptable ou un composé de Zn(+2) et/ou de Cu(+2) correspondant à une concentration en ions métalliques de Zn(+2) et/ou de Cu(+2) comprise entre 100 et 1000 ppm.

2. Composition selon la revendication 1, qui comprend en outre un agent mouillant sélectionné parmi la glycérine, le sorbitol 70%, le PEG-400 et le PEG-600, le propylène glycol ou autres avec une concentration en poids de 5 à 15%.

3. Composition selon la revendication 1 ou 2, qui comprend également un agent tensio-actif non ionique ou amphotérique sélectionné parmi des esters de polyoxyéthylène (monoisostéarate, monoisooléate, monolaurate de sorbitan), des copolymères séquencés de poly(oxypropylène)-poly(oxyéthylène), d'esters de polyhydroxypropyle, d'huile de ricin hydrogénée PEG-40, d'huile de ricin hydrogénée PEG-60 de cocamidopropylbetaïne et autres avec une concentration en poids de 0,2 à 0,8%.

4. Composition selon la revendication 1, 2 ou 3 qui comprend également des fluorures sélectionnés parmi le fluorure de sodium, le fluorhydrate de cétylamine, un dérivé de fluorinate de octadécylamine (DFO) ou une autre source pharmaceutiquement acceptable d'ions fluorures avec une concentration correspondante en ions fluorures allant jusqu'à 2500 ppm.

5. Composition selon l'une des revendications précédentes qui comprend également un édulcorant tel que de la saccharine avec une concentration en poids de 0,005 à 0,10% et/ou du xylitol avec une concentration en poids de 2 à 10%.

6. Composition selon l'une des revendications précédentes qui comprend également des essences avec une concentration en poids de 0,05 à 0,20% et un colorant avec une concentration en poids de 0,0001 à 0,001%.

7. Composition selon l'une quelconque des revendications 1 à 6, pour une utilisation dans le traitement de l'halitose orale.

8. Utilisation de la composition selon l'une quelconque des revendications 1 à 6 afin d'obtenir un bain de bouche pour le traitement de l'halitose orale.

9. Utilisation de la composition selon l'une quelconque des revendications 1 à 6 afin d'obtenir un dentifrice pour le traitement de l'halitose orale.

10. Utilisation de la composition selon l'une quelconque des revendications 1 à 6 afin d'obtenir une poudre dentaire pour le traitement de l'halitose orale.

11. Utilisation de la composition selon l'une quelconque des revendications 1 à 6 afin d'obtenir une pâte à mâcher dentaire pour le traitement de l'halitose orale.
